# EUROPEAN PATENT SPECIFICATION

(11) **EP 2 182 996 B1**
(45) Date of publication and mention of the grant of the patent: **02.01.2013**
(21) Application number: 08796466.4
(22) Date of filing: 23.07.2008
(51) Int. Cl.: A61L 31/02, A61L 31/10, A61L 31/16

(54) **MEDICAL DEVICES COMPRISING POROUS INORGANIC FIBERS FOR THE RELEASE OF THERAPEUTIC AGENTS**
MEDIZINISCHE VORRICHTUNGEN MIT PORÖSEN ANORGANISCHEN FASERN ZUR FREISETZUNG VON THERAPEUTIKA
DISPOSITIFS MÉDICAUX COMPRENANT DES FIBRES INORGANIQUES POREUSES SERVANT À LIBÉRER DES AGENTS THÉRAPEUTIQUES

(30) Priority: 27.07.2007 US 881660
(43) Date of publication of application: 12.05.2010
(73) Proprietor: Boston Scientific Scimed, Inc., Maple Grove, MN 55311-1566 (US)
(72) Inventor: WEBER, Jan, 6228 GJ Maastricht (NL); KOKATE, Jaydeep, Y., Maple Grove, MN 55311 (US); IFTEKAR, Arif, Santa Rosa, CA 95403 (US)
(74) Representative: Hauck Patent- und Rechtsanwälte
(86) International application number: PCT/US2008/070844
(87) International publication number: WO 2009/018037

(56) References cited:
- EP-A- 1 527 754
- WO-A-02/49536
- US-A- 5 024 671
- US-A1- 2005 163 954
- US-A1- 2005 187 605

## Description

### FIELD OF THE INVENTION

The present invention relates to medical devices that release of therapeutic agents.

### BACKGROUND OF THE INVENTION

The *in vivo* delivery of therapeutic agents within the body of a patient is common in the practice of modem medicine. *In vivo* delivery of therapeutic agents is often implemented in conjunction with medical devices that may be temporarily or permanently placed at a target site within the body. These medical devices can be maintained, as required, at their target sites for short or prolonged periods of time, delivering biologically active agents at the target site.

In accordance with certain delivery strategies, a therapeutic agent is provided within or beneath a biostable or biodegradable polymeric layer that is associated with a medical device. Once the medical device is placed at the desired location within a patient, the therapeutic agent is released from the medical device with a profile that is dependent, for example, upon the nature of the therapeutic agent and of the polymeric layer, among other factors.

Examples of such devices include drug eluting coronary stents, which are commercially available from Boston Scientific Corp. (TAXUS), Johnson & Johnson (CYPHER), and others. For example, the TAXUS stent contains a smooth, non-porous polymeric coating consisting of an antiproliferative drug (paclitaxel) within a biostable polymer matrix. The drug diffuses out of the coating over time. Due to the relatively low permeability of paclitaxel within the polymer matrix and due to the fact that the polymer matrix is biostable, a residual amount of the drug may remain in the device beyond its period of usefulness.

Finally, while it is desirable to provide the abluminal surface of the stent with a coating that is capable of releasing an antiproliferative drug to combat restenosis, such a drug may not be equally desirable on the luminal surface of the stent. Moreover, the presence of a polymeric layer on the luminal surface may not be needed for purposes of promoting biocompatibility, as various stent substrate materials, including stainless steel, are known to support endothelial cell growth.

From document EP 1 527 754 A1 a porous medicated stent has become known. The stent has a plurality of pores in the metal which are loaded with medication. The stent may be formed from a porous metal in the form of a wire, tube or metal sheet.

From document US 2005/0187605 A1 a "device and method for producing the device comprising an object, such as a stent, coated with a fibrous polymer" has become known. Electrospinning technology is used to introduce a drug into the fibrous polymer.

From US patent No. 5,024,671 a vascular graft at least partially formed from porous hollow fibers has become known. The porous hollow fibers used to form the vascular grafts further provide a storage situs for temporarily holding a drug or other material for delivery into the blood stream during the healing process.

From document US2005/0163954 A1 medical devices including one or more ceramic fibers have become known.

### SUMMARY OF THE INVENTION

In accordance with an aspect of the invention, implantable or insertable medical devices are provided which comprise a substrate and a porous therapeutic-agent-loaded fiber, wherein said fiber is an inorganic, ceramic fiber connected to a surface of the substrate, characterised in that said fiber is hollow and has at least one open end that is capped with a biodegradable material.

### BRIEF DESCRIPTION OF THE DRAWING

Fig. 1 is a schematic perspective view of a stent that is loosely wrapped with a drug-loaded inorganic fiber, in accordance with an embodiment of the invention.
Figs. 2A, 2B, 3 and 5 are schematic perspective views illustrating some different ways that stents may be associated with drug-loaded porous inorganic fibers, in accordance with various embodiments of the invention.
Figs. 4A and 4B are schematic perspective and cross-sectional views, respectively, of a stent having struts that are covered with drug-loaded porous inorganic fibers, in accordance with an embodiment of the invention.
Figs. 6A through 6C are schematic cross-sectional views illustrating a process of forming a porous inorganic coating that includes porous inorganic fibers, in accordance with an embodiment of the invention.
Fig. 7 contains three schematic perspective views illustrating a process of loading and capping a hollow porous inorganic fiber, in accordance with an embodiment of the invention.
Fig. 8 is a micrograph of a fibrous polystyrene network, in accordance with the prior art.
Fig. 9 is a micrograph of a hollow porous AI2O3 fiber, in accordance with the prior art.
Fig. 10 is a micrograph of a fibrous network of mesoporous T2Os fibers, in accordance with the prior art. Docket No. 06-00197PCT

### DETAILED DESCRIPTION OF THE INVENTION

In accordance with an aspect of the invention, implantable or insertable medical devices are provided which comprise a substrate and a therapeutic-agent-loaded porous inorganic fiber.

The device may comprise, for example, a single inorganic fiber or a plurality of inorganic fibers, for instance, a plurality of distinct fibers or a plurality of fibers that are interconnected within a fibrous network.

The fiber may be, for example, disposed on a surface of the substrate, or it may be disposed within the substrate (e.g., where the substrate is biodegradable).

Medical devices benefiting from the present invention include a variety of implantable or insertable medical devices, which are implanted or inserted into a subject, either for procedural uses or as implants. Examples include stents (including coronary artery stents, peripheral vascular stents such as cerebral stents, urethral stents, ureteral stents, biliary stents, tracheal stents, gastrointestinal stents and esophageal stents), stent grafts, vascular grafts, catheters (e.g., renal or vascular catheters such as balloon catheters), guide wires, balloons, filters (e.g., vena cava filters), vascular access ports, embolization and bulking devices including cerebral aneurysm filler coils (including Guglilmi detachable coils and metal coils), myocardial plugs, pacemaker leads, left ventricular assist hearts and pumps, total artificial hearts, heart valves, vascular valves, tissue engineering scaffolds for cartilage, bone, skin and other in vivo tissue regeneration, cochlear implants, suture anchors, anastomosis clips and rings, tissue staples and ligating clips at surgical sites, cannulae, metal wire ligatures, orthopedic prosthesis such as bone grafts, bone plates, joint prostheses, as well as various other medical devices that are adapted for implantation or insertion into the body and which release a therapeutic agent.

The medical devices of the present invention include implantable and insertable medical devices that are used for systemic treatment, as well as those that are used for the localized treatment of any mammalian tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, ears, spine, nervous system, lungs, trachea, esophagus, intestines, stomach, brain, liver and pancreas, skeletal muscle, smooth muscle, breast, dermal tissue, cartilage, tooth and bone.

As used herein, "treatment" refers to the prevention of a disease or condition, the reduction or elimination of symptoms associated with a disease or condition, or the substantial or complete elimination of a disease or condition. Subjects (also referred to as "patients") include vertebrate subjects such as mammalian subjects, for example, human subjects, pets and livestock.

"Therapeutic agents", "pharmaceuticals," "pharmaceutically active agents", "drugs" and other related terms may be used interchangeably herein.

The substrates with which the inorganic fibers are associated in the practice of the present invention may be biostable or biodegradable. The inorganic fiber may be, for example, disposed on a surface of the substrate (e.g., wrapped around, interwoven with or attached to the substrate) or it may be disposed within the substrate (e.g., embedded within a biodegradable substrate).

As defined herein, a "biostable" material is one which remains intact over the time period that the medical device is intended to remain implanted within the body. Similarly, as defined herein, a "biodegradable" material is one which does not remain intact over the period which the medical device is intended to remain within the body, for example, due to any of a variety of mechanisms including dissolution, chemical breakdown, and so forth, of the region. Depending upon the device within which the biodegradable material is disposed and the mechanism of degradation of the biodegradable material, this period may vary, for example, from less than or equal to I hour to 3 hours to 12 hours to 1 day to 3 days to 1 week to I month to 3 months to 1 year or longer.

Material for forming the substrate thus include various organic materials (i.e., materials containing one or more types or organic species), such as polymers and various inorganic materials (i.e., materials containing one or more inorganic species), such as metallic materials (e.g., metals and metal alloys) and non-metallic materials (e.g., carbon, semiconductors, glasses, and ceramics containing various metal- and non-metal-oxides, various metal- and non-metal-nitrides, various metal- and non-metal-carbides, various metal- and non-metal-borides, various metal- and non-metal-phosphates, and various metal- and non-metal-sulfides, among others).

Specific examples of non-metallic inorganic materials may be selected, for example, from materials containing one or more of the following: metal-based ceramics including aluminum oxides and transition metal oxides (e.g., oxides of titanium, zirconium, hafnium, tantalum, molybdenum, tungsten, rhenium, and iridium); semi-metal-based ceramics, such as those containing silicon, silicon oxides (sometimes referred to as glass ceramics), germanium oxides, silicon and germanium nitrides, silicon and germanium carbides, calcium phosphate ceramics (e.g., hydroxyapatite); and carbon and carbon-based, ceramic-like materials such as carbon nitrides, among many others.

Specific examples of metallic inorganic materials may be selected, for example, from substantially pure metals (e.g., biostable metals such as gold, platinum, palladium, iridium, osmium, rhodium, titanium, tantalum, tungsten, and ruthenium, and biodegradable metals such as magnesium, zinc and iron), metal alloys comprising iron and chromium (e.g., stainless steels, including platinum-enriched radiopaque stainless steel), alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N) and alloys comprising cobalt, chromium, tungsten and nickel (e.g., L605), alloys comprising nickel and chromium (e.g., inconel alloys), and alloys comprising magnesium and/or iron.

Further examples of metallic substrate materials include the biodegradable metallic materials described in U.S. Patent App. Pub. No. 2002/0004060 A1, entitled "Metallic implant which is degradable in vivo." These include substantially pure metals and metal alloys whose main constituent is selected from alkali metals, alkaline earth metals, iron, and zinc, for example, metals and metal alloys containing magnesium, iron or zinc as a main constituent and one or more optional additional constituents selected from the following: alkali metals such as Li, alkaline-earth metals such as Ca and Mg, transition metals such as Mn, Co, Ni, Cr, Cu, Cd, Zr, Ag, Au, Pd, Pt, Re, Fe and Zn, Group IIIa metals such as A1, and Group IVa elements such as C, Si, Sn and Pb.

Examples of polymeric substrate materials include a variety of biostable and biodegradable polymers, with biodegradable polymers being preferred in certain embodiments, particularly those in which the inorganic fiber is embedded in the substrate material. Fibers may also be embedded in a biodegradable polymer coating that is disposed over a substrate.

Examples of biodegradable polymers for use in the present invention may be selected from suitable members of the following, among many others: (a) polyester homopolymers and copolymers such as polyglycolide (PGA), polylactide (PLA) including poly-L-lactide, poly-D-lactide and poly-D,L-lactide, poly(beta-hydroxybutyrate), poly-D-gluconate, poly-L-gluconate, poly-D,L-gluconate, poly(epsilon-caprolactone), poly(delta-valerolactone), poly(p-dioxanone), poly(trimethylene carbonate), poly(lactide-co-glycolide) (PLGA), poly(lactide-co-delta-valerolactone), poly(lactide-co-epsilon-caprolactone), poly(lactide-co-beta-malic acid), poly(lactide-co-trimethylene carbonate), poly(glycolide-co-trimethylene carbonate), poly(beta-hydroxybutyrate-co-beta-hydroxyvalerate), poly[1,3-bis(p-carboxyphenoxy)propane-co-sebacic acid], and poly(sebacic acid-co-fumaric acid), among others, (b) poly(ortho esters) such as those synthesized by copolymerization of various diketene acetals and diols, among others, (c) polyanhydrides such as poly(adipic anhydride), poly(suberic anhydride), poly(sebacic anhydride), poly(dodecanedioic anhydride), poly(maleic anhydride), poly[1,3-bis(p-carboxyphenoxy)methane anhydride], and poly[alpha,omega-bis(p-carboxyphenoxy)alkane anhydrides] such as poly[1,3-bis(p-carboxyphenoxy)propane anhydride] and poly[1,3-bis(p-carboxyphenoxy)hexane anhydride], among others; and (d) amino-acid-based polymers including tyrosine-based polyarylates (e.g., copolymers of a diphenol and a diacid linked by ester bonds, with diphenols selected, for instance, from ethyl, butyl, hexyl, octyl and benzyl esters of desaminotyrosyl-tyrosine and diacids selected, for instance, from succinic, glutaric, adipic, suberic and sebacic acid), tyrosine-based polycarbonates (e.g., copolymers formed by the condensation polymerization of phosgene and a diphenol selected, for instance, from ethyl, butyl, hexyl, octyl and benzyl esters of desaminotyrosyl-tyrosine), and tyrosine-, leucine- and lysine-based polyester-amides; specific examples of tyrosine-based polymers include includes polymers that are comprised of a combination of desaminotyrosyl tyrosine hexyl ester, desaminotyrosyl tyrosine, and various di-acids, for example, succinic acid and adipic acid, among others.

As used herein, a "nanopore" is a pore having a width that does not exceed I micron in width. As used herein, "micropores" are smaller than 2 nm in width, "mesopores" range from 2 to 50 nm in width, and "macropores" are larger than 50 nm in width.

As used herein a "porous" fiber is a fiber that contains pores. A "nanoporous fiber" is a fiber that contains nanopores; a "macroporous fiber" is a fiber that contains macropores; and so forth.

As used herein, an "inorganic fiber" is a fiber that contains one or more inorganic materials, such as those described above in conjunction with substrate materials, for example, in an amount ranging from 50 wt% or less to 75 wt% to 90 wt% to 95 wt% to 97.5 wt% to 99 wt% or more inorganic materials.

As used herein, a "ceramic fiber" is a fiber that contains one or more ceramic materials, such as those described above in conjunction with substrate materials, for example, in an amount ranging from 50 wt% or less to 75 wt% to 90 wt% to 95 wt% to 97.5 wt% to 99 wt% or more ceramic materials.

As used herein, a "metallic fiber" is a fiber that contains one or more metals, such as those described above in conjunction with substrate materials, for example, in an amount ranging from 50 wt% or less to 75 wt% to 90 wt% to 95 wt% to 97.5 wt% to 99 wt% or more metals.

As used herein, a "sol-gel derived ceramic fiber" is one that is formed using sol-gel chemistry.

It is known that certain ceramic materials are bioactive. As defined herein a "bioactive material" is a material that adheres to adjacent tissue or that fosters cell adhesion and/or proliferation (with associated tissue coverage), for example, bone tissue or soft tissue such as endothelial tissue, with minimal adverse biological effects (e.g., the formation of connective tissue such as fibrous connective tissue). Examples of bioactive ceramic materials, sometimes referred to as "bioceramics," include calcium phosphate ceramics, for example, hydroxyapatite; calcium-phosphate glasses, sometimes referred to as glass ceramics, for example, bioglass; and various metal oxide ceramics such as titanium oxide. In this regard, it has been proposed that the formation of bone-like apatite on artificial materials is induced by functional groups, including Si-OH, Ti-OH, Zr-OH, Nb-OH and Ta-OH, among others. T. Kokubo et al., "Novel bioactive materials with different mechanical properties," Biomaterials, 2003, 24(13): 2161-75.

It is also known that bioactivity depends upon the structure of a given surface. See, e.g., the review by E.K.F Yim et al., "Significance of synthetic nanostructures in dictating cellular response," Nanomedicine: Nanotechnology, Biology, and Medicine 1 (2005) 10-21, which reports that smooth muscle cells and endothelial cells have improved cell adhesion and proliferation on nanopattemed surfaces. Both types of cells were sensitive to nanotopography. Yim et al. report improved adhesion and growth for endothelial cells on a substrate with 13 nm high islands relative to 35 and 95 nm high islands. Endothelial cells were also susceptible to surface chemistry. See also, e.g., Viitala R. et al., "Surface properties of in vitro bioactive and non-bioactive sol-gel derived materials," Biomaterials. 2002 Aug; 23(15): 3073-86.

Certain materials are also known to have excellent hemocompatibility. Titanium oxide films containing tantalum, Ti(Ta⁺⁵)O₂ have been reported to exhibit attractive blood compatibility including and overall excellent antithrombogenic properties (as characterized by clotting time, platelet adhesion measurement and in vivo experiments), which have been hypothesized to be related to physical properties such as surface energy and semiconductivity. J.Y. Chen et al., "Antithrombogenic investigation of surface energy and optical bandgap and hemocompatibility mechanism of Ti(Ta+5)O2) thin films," Biomaterials 23 (2002) 2545-2552. See also, for example, N. Huang et al., "Hemocompatibility of titanium oxide films," Biomaterials 24 (2003) 2177-2187.

Titanium oxide fibers containing tantalum may be formed, for example, by forming a titanium oxide fiber, followed by the use of a plasma immersion process to create Ta doping within the fibers. As another example, metallic fibers may be formed from titanium and tantalum, after which the titanium/tantalum hybrid fibers are oxidized in air as described in J.Y. Chen et al. to form Ti(Ta⁺⁵)O₂.

Inorganic fibers for use in the invention can vary widely in width (e.g., diameter for a fiber with circular diameter), ranging, for example, from 1000 microns or more to 500 microns to 100 microns to 50 microns to 10 microns to 5 microns to 1 micron to 0.5 micron (500 nm) to 0.1 micron (100 nm) to 0.05 micron (50 nm) to 0.025 micron (25 nm), or less.

As used herein a "nanofiber" is one that is less than I micron in width.

In some embodiments of the invention, the inorganic fiber is a linear (non-branched) fiber. In some embodiments, the inorganic fiber is part of a fibrous network.

In the invention, the inorganic fiber is a solid hollow inorganic fiber. For example, the hollow fiber may have a hollow core, in which case the core may be filled with a therapeutic agent, either alone or admixed with another material, for instance, a polymeric material (e.g., one of the biodegradable polymers set forth above, among many other possibilities). By capping the ends of such fibers, the release of the therapeutic agent is regulated by the porous walls of the fiber (and by the presence of other optional materials that may be admixed with the therapeutic agent, such as polymers). Therapeutic agent loading in such embodiments is precise, as it can be calculated based on the diameter of the hollow core.

Depending on the embodiment, the capping material may be biostable or biodegradable. Where the capping material is biodegradable, upon degradation of the capping material, the release of any drug remaining in the core at that time will be accelerated.

As noted above, in the present invention, the inorganic fiber(s) may be associated with the substrate in a variety of ways. For example, the inorganic fiber(s) may be embedded within the substrate. Inorganic fibers may also be associated with the substrate by means of a variety of suitable fiber-based fabrication techniques including, for example, various woven and non-woven techniques (e.g., by knitting or braiding with substrate elements, winding or wrapping around substrate elements, spraying onto substrate elements, etc.). In certain embodiments, the fibers may be adhered to the substrate, to each other, or both, by various techniques, including thermal fusion, adhesive binding or simple mechanical entanglement, among others.

In certain embodiments, the inorganic fibers are associated with a tubular substrate, for example, a stent or another tubular medical device substrate. For example, the fibers may be in the form of a woven or non-woven tubular construction that is concentric with the tubular substrate, the fibers may be interwoven with elements of the tubular substrate (e.g., filaments or stent struts, which give a stent its mechanical integrity), or the fibers may be adhered to elements of the tubular substrate, among other possible methods of association. As an example of the latter, one may simply connect inorganic fibers to an outer stent surface using dots of biodegradable polymer as an adhesive.

In certain embodiments, the inorganic fibers are associated with an expandable substrate, for example, a balloon-expandable or self-expanding stent, or another expandable medical device substrate.

In some cases, the inorganic fibers are associated with the substrate (e.g., formed on the substrate, applied to the substrate, etc.) while in an expanded state, after which the substrate is contracted prior to delivery to a patient.

In other cases, the inorganic fibers may be associated with the substrate while in a contracted state. For example, in certain of these embodiments, the inorganic fiber(s) are in the form of a woven or non-woven construction that is concentric with the tubular substrate but which allows for expansion. For instance, the fiber may be loosely wrapped around the device, the fiber may formed into a woven or non-woven tube (or a woven or non-woven sheet of inorganic fibers may be wrapped into the shape of a tube and the ends joined) that is concentric with the device, and so forth.

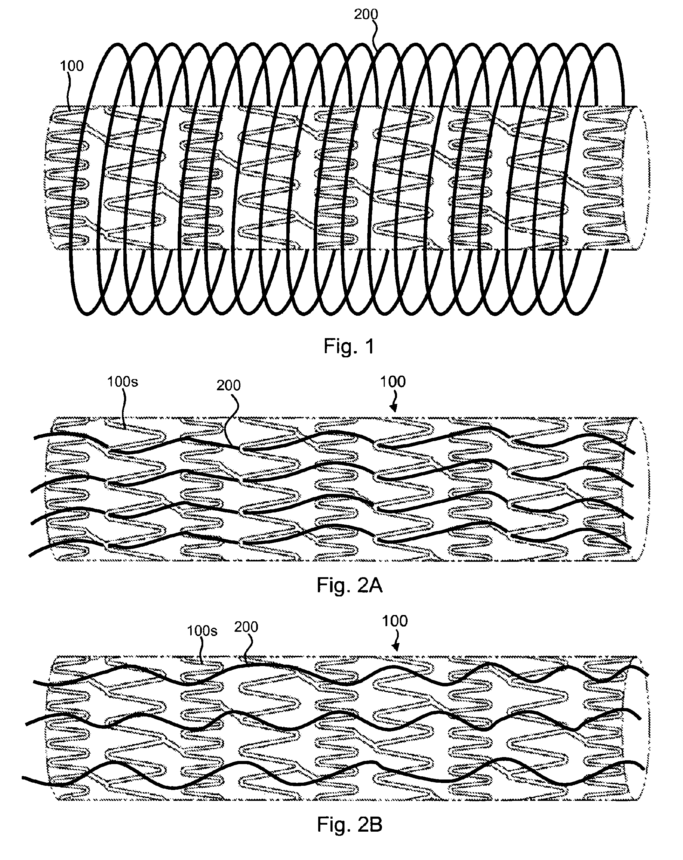

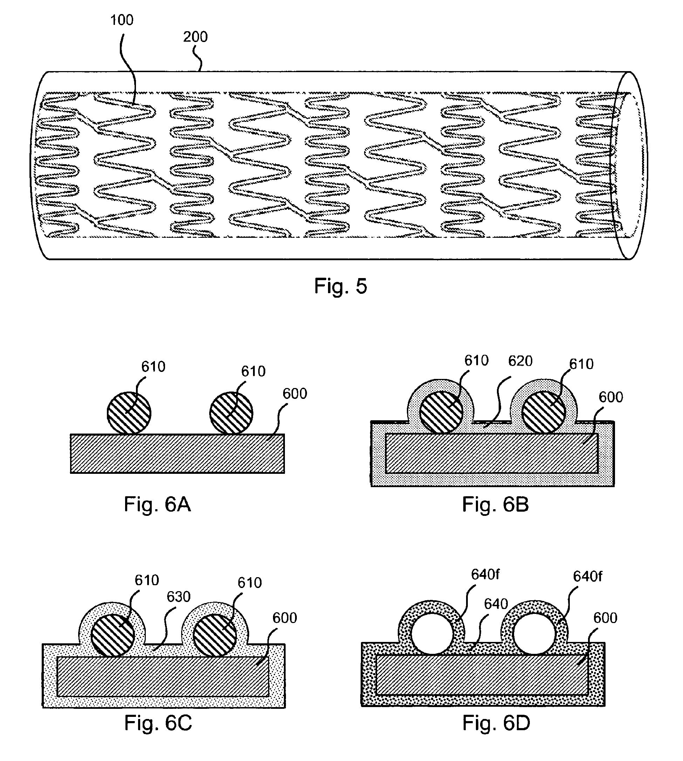

For example, a fiber 200 may be loosely wrapped around an expandable stent 100, as schematically illustrated in Fig. 1. Fig. 5 illustrates an embodiment in which a woven or non-woven tubular construction 200 is disposed around a stent 100. Note that in these embodiments, the fibers may be ultimately pinned between the stent and the lumen wall once the stent expanded in vivo. Such embodiments may be useful, for example, for purposes of maintaining the mechanical integrity of the blood vessel at the site of a balloon angioplasty procedure, for coverage of an aneurism in a blood vessel wall, for coverage of vulnerable plaque, and so forth.

In certain other embodiments, the fibers are more closely associated with the substrate, for instance, being interwoven with or attached to elements of the substrate (e.g., stent filaments, stent struts, etc., which give a stent its mechanical integrity).

For example, multiple fibers 200 may be woven through the struts 100s of an expandable stent 100, as illustrated in Fig. 2A. The fibers 200 are woven longitudinally, rather than radially, in the embodiment shown. In alternative embodiment shown in Fig. 2B, the fibers 200 are longitudinally disposed along the outer surface of the stent without interweaving the fibers with the stent struts 100s, for example, by adhering the fibers to the outer surface of the stent struts 100s a various points along the length of the stent.

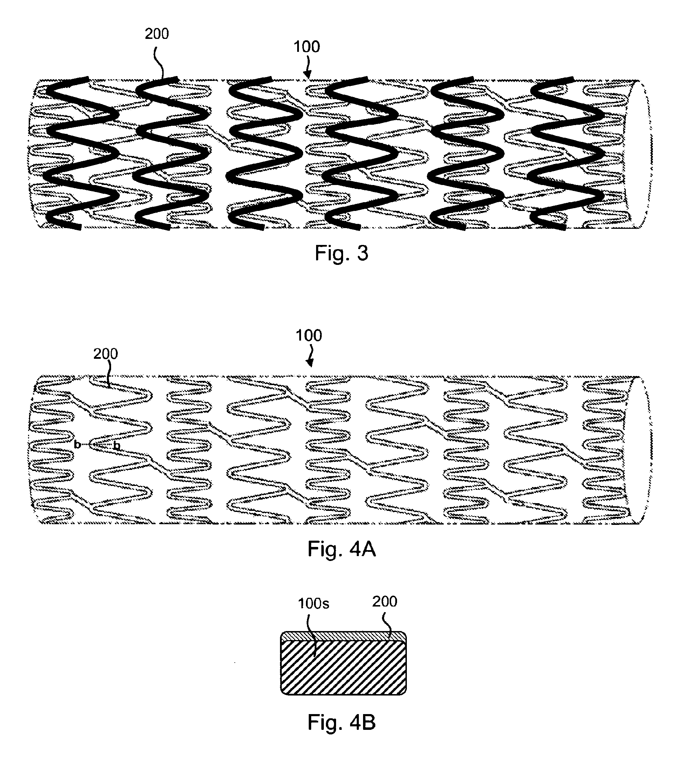

In other embodiments, the fibers are wrapped around the substrates in ways that allow for expansion of the substrate. For example, in the case of a tubular substrate that expands radially, the fiber may be wrapped around the tubular substrate such that the fiber extends distally, then proximally, then distally again, then proximally again, and so forth as it circles the substrate. Fig. 3 illustrates such an embodiment in which fibers 200 are wrapped around the stent 100 in a zigzag configuration.

In other embodiments, inorganic fibers are applied to individual elements of the expandable device. For example, fibers 200 may be applied to or formed on the outer surface of a stent 100 such as that shown in Fig. 4A. Fig 4B is an expanded cross-section taken along line b--b of Fig. 4A and illustrates the fibrous layer 200, disposed over an underlying stent strut 100s. Such a structure may be formed, for example, by adhering individual fibers to the struts, by adhering a woven or non-woven tubular construction (e.g., a wrapped sheet) to the stent and then removing those portions overlying the openings between the stent struts, by electrospraying a fibrous layer on the struts, and so forth. Because the fibers are found on the struts only, and because the struts themselves do not lengthen significantly during stent expansion, large strains (and thus stresses) on the fibrous layer are avoided.

Porous inorganic fibers may be formed in various ways and, as indicated above, they may be formed first and then applied to a substrate, or they may be formed directly on a substrate.

As an example of the latter family of techniques, porous inorganic fibers may be spun onto a substrate (e.g., a stationary substrate, a rotating substrate, etc.) either as individual fibers or as a fibrous network. The porous inorganic fibers themselves may be solid or hollow in nature.

In certain of these embodiments, electrostatic spinning processes may be employed. Electrostatic spinning processes have been described, for example, in Annis et al. in "An Elastomeric Vascular Prosthesis", Trans. Am. Soc. Artif. Intern. Organs, Vol. XXIV, pages 209-214 (1978), U.S. Patent No. 4,044,404 to Martin et al., U.S. Patent No. 4,842,505 to Annis et al., U.S. Patent No. 4,738,740 to Pinchuk et al., and U.S. Patent No. 4,743,252 to Martin Jr. et al. In electrostatic spinning, electrostatic charge generation components are employed to develop an electrostatic charge between the distributor (e.g., a spinneret) and a target, for example, a rotating substrate. For example, target may be grounded or negatively charged, while the distributor is positively charged. Alternatively, the distributor may be grounded or negatively charged, while the target can be positively charged. The potential that is employed may be constant or variable. As a result of the electrostatic charge that is generated, the fibers experience a force that accelerates them from the distributor to the target.

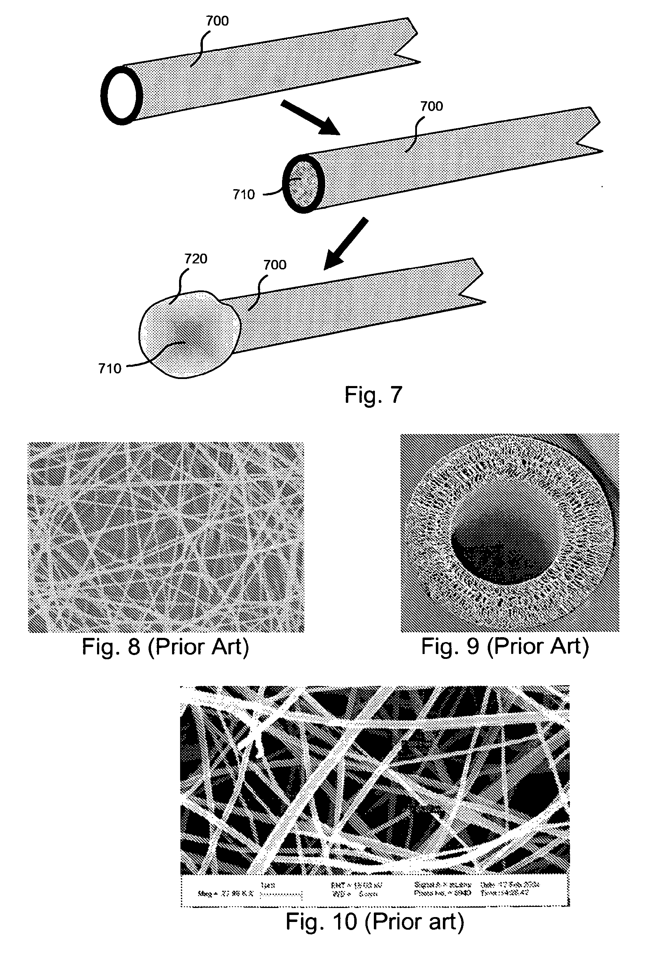

As a specific example, M. Macias et al., Microporous and Mesoporous Materials 86 (2005) 1-13 describe the production of mesoporous metal oxide ceramic fibers, including TiO₂, Ta₂O₅ and TaNbO₅, with diameters under 1 micron. The fibers were electrospun as a non-woven mesh (or paper) from a fiber precursor gel that further contained a structure-directing organic agent (i.e., a surfactant). The structure-directing organic agent was subsequently removed by heating to elevated temperatures. P. Viswanathamurthi et al., Chemical Physics Letters 374 (2003) 79-84, describe similar methods for producing porous niobium oxide fibers. A fibrous network of Ta₂O₅ fibers are shown in Fig. 10 (from Macias et al.).

In accordance with an embodiment of the invention, such fibers may be directly electrospun on a medical device substrate (e.g., a rotating stent). In another embodiment, the fibers may be spun onto a flat substrate forming a so-called nano-paper, which may then be applied to a medical device substrate. In another embodiment, the fibers may be spun onto a sheath.

Other methods for forming medical devices in accordance with the invention utilize polymer fibers as templates for the creation of hollow porous inorganic fibers. For example, polymer fibers (e.g., polystyrene fibers, among many others) may be first be applied to a medical device substrate (e.g., by directly spinning the fibers on the substrate or by applying previously formed fibers to the substrate). Such a structure is illustrated schematically in Fig. 6A which shows a medical device substrate 600 (e.g., a stent strut) and two polymer fibers 610 in cross section.

T. Lin et al. "The charge effect of cationic surfactants on the elimination of fibre beads in the electrospinning of polystyrene," Nanotechnology 15 (2004) 1375-1381 and T. Jarusuwannapoom et al., "Effect of solvents on electro-spinnability of polystyrene solutions and morphological appearance of resulting electrospun polystyrene fibers," European Polymer Journal 41 (2005) 409-421 describe techniques for electrospinning polystyrene fibers with various textures and sizes (diameters typically on the order of 100 nm to 1 micron). An SEM of a polystyrene fiber network is shown in Fig. 8 (from Lin et al.).

For example, in an embodiment of the present invention, such processes may be used to create polystyrene fibers on a stent that is held at a suitable potential and rotated during electrospinning, thereby covering the stent in a fibrous network of polystyrene fibers.

Fiber coated substrates formed using these or other techniques may then be used as templates for the creation of inorganic hollow fibers. For example, hollow ceramic fibers may be formed using a combination of layer-by-layer and sol-gel processing techniques. This combination of processes has been successfully employed to produce hollow LiNbO₃ spheres. See Colloids and Colloids assemblies, Frank Caruso Ed., Wiley-VCH, ISBN 3-527-30660-9, pp. 266-269.

By way of background, it is well known that ceramic regions may be formed using sol-gel processing. In a typical sol-gel process, precursor materials, typically selected from inorganic metallic and semi-metallic salts, metallic and semi-metallic complexes/chelates, metallic and semi-metallic hydroxides, and organometallic and organo-semi-metallic compounds such as metal alkoxides and alkoxysilanes, are subjected to hydrolysis and condensation reactions in the formation of ceramic materials. Commonly, an alkoxide of choice (e.g., a methoxide, ethoxide, isopropoxide, *tert-*butoxide, etc.) of a semi-metal or metal of choice (e.g., silicon, germanium, aluminum, zirconium, titanium, tin, iron, hafnium, tantalum, molybdenum, tungsten, rhenium, iridium, etc.) is subjected to hydrolysis and condensation in the formation of ceramic regions.

Also well known is the "layer-by-layer" (LBL) method, by which multilayer coatings are formed on substrates via electrostatic self-assembly of charged materials. In the LBL method, a first layer having a first surface charge is typically deposited on an underlying substrate (e.g.., a medical device or portion thereof), followed by a second layer having a second surface charge that is opposite in sign to the surface charge of the first layer, and so forth. The charge on the outer layer is reversed upon deposition of each sequential layer. Commonly, 5 to 10 to 25 to 50 to 100 to 200 or more layers are applied in this technique, depending on the desired thickness. LBL techniques commonly employ charged polymer species known as "polyelectrolytes". Commonly, the number of charged groups is so large that the polymers are soluble in polar solvents (including water) when in ionically dissociated form (also called polyions). Depending on the type of dissociable groups, polyelectrolytes may be classified as polyacids and polybases. When dissociated, polyacids form polyelectrolyte anions (also known as polyanions), with protons being split off. Specific examples of polyacids/polyanions include poly(styrenesulfonate) (e.g., poly(sodium styrene sulfonate) (PSS)), polyacrylic acid, polyvinylsulfate, polyvinylsulfonate, sodium alginate, eudragit, gelatin, hyaluronic acid, carrageenan, chondroitin sulfate and carboxymethylcellulose, among many others. Polybases contain groups which are capable of accepting protons, e.g., by reaction with acids, with a salt being formed. By accepting protons, polybases form polyelectrolyte cations (also known as polycations). Specific examples of polybases/polycations include protamine sulfate, poly(allylamine) (e.g., poly(allylamine hydrochloride) (PAH)), polydiallyldimethylammonium species, polyethyleneimine (PEI), polyvinylamine, polyvinylpyridine, chitosan, gelatin, spermidine and albumin, among many others.

Once polymer fibers 610 are positioned on a suitable substrate 600, for example, as shown schematically in Fig. 6A, the fiber-covered substrate is then exposed to a series of polycation and polyanion solutions, thereby forming a multilayer polyelectrolyte coating over the structure. A structure of this type is illustrated schematically in Fig. 6B which shows the medical device substrate 600, polymer fibers 610, and multilayer polyelectrolyte coating 620. Note that both the substrate 600 and the fibers 610 are covered by the polyelectrolyte coating 620. Hence, the polyelectrolyte coating 620 acts to connect the substrate 600 to the fibers 610.

In a next step, a sol-gel-type process is carried out within the polyelectrolyte layers. First, the structure of Fig. 6B is washed in an anhydrous solvent, for example, an anhydrous alcohol. This removes essentially all the water from the structure, except of the water that remains adsorbed within the polyelectrolyte coating 620. The structure is then immersed in an anhydrous sol-gel precursor solution (e.g. a solution of a semi-metal or metal alkoxide in anhydrous alcohol). As the precursor infiltrates the polyelectrolyte coating 620, the presence of water in the coating 620 leads to the hydrolysis and condensation of the precursor (i.e., a sol-gel reaction takes place), resulting in polyelectrolyte/ceramic hybrid coating 630 as illustrated in Fig. 6C. The thickness of the coating 630 will vary with the number of polyelectrolyte layers in the polyelectrolyte coating 620, with more layers leading to thicker coatings. For further information on layer-by-layer/sol-gel processing, see, e.g., Colloids and Colloid Assemblies, Wiley-VCH, edited by Frank Caruso, ISBN 3-527-30660-9, pp. 266-269.

Finally, the structure of Fig. 6C is heated to elevated temperature (e.g., 400-600°C) to remove the polymeric constituents of the structure. As a result, a porous ceramic coating 640 is formed on the substrate 600 as illustrated in Fig. 6D. The ceramic coating 640 includes numerous hollow ceramic fibers 640f. Assuming that the fibers 610 are completely immersed during the layer-by-layer and sol-gel processing, the ends of the fibers will be closed.

One advantage of a process of this type is that the hollow fiber network is connected to the substrate surface. Another advantage is that the fiber network can be located on one surface of the substrate, but not another. For example, the hollow fiber network can be located only on the outer abluminal surface of a stent. To achieve this, one may remove portions of the electrospun polymer fiber network (e.g., from the inner surface of the stent, from the windows between the stent struts, etc.) before applying the LBL coating. After sol-gel processing and calcination, those portions of the substrate from which the fiber has been removed will have less capacity to act as a therapeutic agent reservoir, for example, because no hollow fibers are present and because the ceramic layer that is formed typically has less surface area due to the absence of the fibers.

In some embodiments, certain portions of the substrate/fiber assembly may be provided with more polyelectrolyte layers than others, such that the final ceramic layer is thicker at some locations than others. For example, one end of a stent/fiber assembly may be dipped in more polyelectrolyte solutions than the other end, leading to a ceramic layer that is thicker on one end of the stent, which will have an effect on drug release.

In some embodiments, after providing a first fiber network and after providing a first multilayer coating, an additional fiber network is formed over the first one. The additional fiber network is then provided with its initial multilayer coating, during which the underlying fiber network will be provided with further polyelectrolyte layers. This structure is then subjected to sol-gel processing and calcination, forming a ceramic fiber network with two types of ceramic fibers (i.e., thin-walled fibers and thick-walled fibers).

An advantage of using electrospinning techniques such as those described in Lin et al. and Jarusuwannapoom et al., *supra,* is that very thin fibers (e.g., I micron or less) may be formed. As a result, the ceramic fibers formed using these fibers as templates will also be very thin, allowing them to be deformed (e.g., bent) to a substantial degree without cracking.

Analogous processing can be conducted on polymer fibers that are formed using fiber spinning processes in which continuous fibers are spun. These processes typically employ extrusion nozzles having one or more orifices, also called distributors, jets, or spinnerets. Fibers having a variety of cross-sectional shapes may be formed, depending upon the shape of the orifice(s). Some examples of fiber cross-sections include polygonal (e.g., triangular, rectangular, hexagonal, etc.), circular, oval and multi-lobed cross-sections, among others. Fiber diameters produced by such techniques vary widely, for example, ranging from 100's of nanometers to 100's microns in width. In melt spinning processes, polymers are heated to melt temperature prior to extrusion. In wet and dry spinning processes, polymers are dissolved in a solvent prior to extrusion. Regardless of the technique, the resulting fiber is generally taken up on a rotating mandrel or another take-up device.

Such fibers can be subjected to LBL processing, sol-gel processing and calcination prior to or after association with a medical device substrate. Where such processing is conducted before association with a medical device substrate, a ceramic layer will not be formed on the substrate, as described above in conjunction with Figs. 6A-6D (see in particular Fig. 6D). Nonetheless, the fibers can be associated with the substrate in a variety of ways, various examples of which are discussed above, among others.

In some embodiments of the invention, fibers may be spun onto a rotating substrate (e.g., a medical device substrate or a temporary substrate) where they become bonded to one another. For instance, a polymer solution (or melt) can be extruded from a spinneret, thereby forming a plurality of filaments which are wound onto a rotating mandrel or medical device, as the spinneret reciprocates relative to the mandrel or device. The drying (or cooling) parameters may be controlled such that some residual solvent (or tackiness) remains in the filaments as they are wrapped upon the mandrel or device. Upon further solvent evaporation (or cooling), the overlapping fibers on the mandrel or device become bonded to each other. Complex hollow fiber network structures may be formed, for example, using a tapered or stepped mandrel or using a mandrel that can be dissolved, melted, deflated or other otherwise reduced in size for removal after the structure is formed. The resulting fiber network, can then be subjected to LBL processing, sol-gel processing and calcination as described above.

As previously indicated, where the fibers are completely immersed during the layer-by-layer and sol-gel processing, the ends of the fibers will be closed. Open ended fibers, on the other hand, can be formed by dipping the fiber (either with or with out an associated medical device substrate) only partially into the LBL polyelectrolyte solutions, the sol-gel precursor solution, or both. For example, a stent with a fiber coating can be dipped into the LBL polyelectrolyte solutions in a way such that one end is not exposed to these solutions. This structure is then dipped into a sol-gel precursor solution such that the opposite end is not exposed to the precursor solution. This results in a structure in which each end has been denied a critical processing step (i.e., either LBL processing or a sol-gel processing). Calcination will create open ended fibers on each end of the stent.

Of course, processes other than the above can be used to generate hollow porous inorganic fibers for use in the present invention.

For example, processes other than LBL/sol-gel processing can be used in conjunction with fiber templates such as those described in the preceding paragraphs. In one embodiment, charged particles may be accelerated onto the fibers (where they become fused to one another) by subjecting them to an electric field. For example, one can place the fibers (e.g., organic fibers) in between the source of the charged particles and a grounded plate, such that the particles will form a film on the fibers by collision/fusion on their way to the grounded plate. If desired, the trajectory of the particles may be further influenced through the use of a secondary electric field or a magnetic field, where desired. In this way, a porous structure surrounding the fibers may be formed from the fused particles. Where the particles are magnetic or ferromagnetic particles, they may be accelerated onto the fibers by subjecting them to a suitable magnetic field, with the trajectory of the particles being further influenced, if desired, through the use of a secondary magnetic field. In general, such techniques are performed in a vacuum environment. As a specific example, a system for performing such a deposition is available from Mantis Deposition Ltd., Thame, Oxfordshire, United Kingdom, who market a high-pressure sputtering source which is able to generate particles from a sputter target with as few as 30 atoms up to those with diameters exceeding 15nm. Systems like the Mantis Deposition Ltd. system can produce particles, the majority of which (approximately 80%) have a charge of one electron. Consequently, a magnetic field or a secondary electric field can be used to separate particles of similar weight from one another (because lighter particles are deflected to a greater degree in a given field than are the larger particles of the same charge). For example, the above Mantis Deposition Ltd. system is able to produce charged particle streams with a very narrow mass distribution. A system similar to the Mantis system can be obtained from Oxford Applied Research, Witney, Oxon, UK. Using these and similar systems, thin nanoporous metallic layers may be deposited on a variety of fibers. Such processes are room temperature processes. Thus one may, for example, electrospin therapeutic-agent containing fibers (e.g., fibers of a biostable polymer such as poly[styrene-*b*-isobutylene-b-styrene] or a biodegradable polymer such as PLGA that contain a therapeutic agent such as paclitaxel, sirolimus, everolimus, tacrolimus or zotarolimus, among many other possibilities) and cover them with a nanoporous metal coating. As another example, one can deposit a nanoporous metal coating on fibers formed of one or more removable polymers (e.g., polystyrene) and then remove (e.g., burn off, dissolve, etc.) the polymer core, after which one may fill the core with a therapeutic agent. In this way, porous metallic fibers may be formed, including those formed from biostable metals, biodegradable metals or both.

In conjunction with another example, it is noted that systems such as the above system from Mantis Deposition Ltd. allows one to create non-porous layers of metal particles on a surface, simply by increasing the acceleration voltage to such an extent that the particles fuse/melt fully together. By depositing a metal coating using biodegradable metal particles such as magnesium, zinc or iron particles (which may be optionally codeposited with non-biodegradable metal particles) a coating is created that becomes porous as a result of biodegradation in the body. Depositing such a coating on a drug-filled fiber (e.g., a drug-loaded biostable polymer fiber, a drug- loaded biodegradable polymer fiber, etc.) allows one to create a drug filled inorganic fiber that becomes porous in vivo and that is at least partially biodegradable in vivo.

As another example, Fraunhofer Gesellschaft has developed a process for producing hollow ceramic fibers for use in capillary membranes. In this process, cellulose is dissolved in a mixture of an organic solvent and water and a ceramic powder is suspended in the solution. This mass is extruded into a water bath through an annular nozzle while a water jet is injected through an opening in the nozzle center. Upon contact with water, a stable hollow fiber, which contains the suspended ceramic powder, is formed. These fibers are collected on reels, dried and sintered in air. During the sintering the cellulose matrix bums out leaving behind a structure with porous walls. One such fiber, an Al₂O₃ fiber having a diameter of 1.1 mm, is shown in Fig. 9.

Therapeutic agent can be loaded through the porous wall of a closed-end hollow fiber or into a porous non-hollow fiber, by exposure to solutions that contain a therapeutic agent of interest. Loading can be enhanced by using solutions with high therapeutic agent concentrations, by loading at elevated temperature, by prolonged exposure to the therapeutic-agent containing solution, by exposure to therapeutic agent containing supercritical fluids such as carbon dioxide or nitric-oxide (with or without co-solvents such as methanol or ethanol), by exposure to the therapeutic-agent containing solution in a high pressure environment to drive the solution into the fibers (with or without pressure cycling to remove the solvent and allow further solution to penetrate the fiber), or combinations of one or more of these measures, among others.

For open ended hollow fibers, and with reference to Fig. 7, therapeutic agent 710 can be loaded into the fibers 700 using similar processing techniques. Alternatively, particularly for larger diameter fibers, a solution or melt that contains the therapeutic agent, and optionally a polymer, can be loaded into the hollow fibers by more direct techniques, including, for example, introduction using a micro-pipette. Once loaded, the open ends of the fibers may be capped, for example, by dipping the ends into a solution or melt of a capping material, thereby forming a cap 720 over the ends. The cap 720 may be a biostable or biodegradable. In either case, drug elution is controlled, at least initially, by the porosity of the fiber walls (and will also be affected by any polymer that is admixed with the therapeutic agent). In the case of a biodegradable cap, a more rapid release of any remaining drug will ensue, once the cap dissolves. Such release may approach 100%.

In accordance with another technique, open ended hollow fibers are first placed in a closed beaker with an inlet and an outlet. A vacuum is applied to the outlet to remove the air from within the fibers. The outlet to the vacuum pump is then closed and the inlet is opened to fill the beaker with a solution containing the drugs. The solution will also enter interior of the fibers. After solvent evaporation, a drug-containing layer exists on both the interior as well as the exterior of the fibers. This process may be repeated multiple times to fill the fibers as much as possible. In order to remove the drug-containing layer from the outside of the fibers, without removing the same from the inside, one can flush the fibers in a solvent bath. The drug-containing layer on the outside of the fibers is exposed directly to the solvent, whereas the drug on the interior has to pass through the ends of the fibers. A small amount of drug will be removed from the ends, however, one can simply cut off a piece of the fiber ends to assure that the remaining piece is filled, if desired. Alternatively, the fibers may be dipped in a polymer to close both ends, followed by washing of the outside (e.g., in a solvent that doesn't dissolve the polymer).

In one exemplary embodiment of the invention, a stainless steel vascular stent is formed which comprises fibers that are loaded with a therapeutic agent, for example, an antiproliferative agent such as paclitaxel, sirolimus, everolimus, tacrolimus or zotarolimus, among many other possibilities. While it is useful to release such an agent from the abluminal surface of the stent to reduce restenosis, such a drug may not be equally useful on the luminal surface of the stent. Therefore, in this embodiment, inorganic fibers are provided only on the outer abluminal surface of the stent that engages the vessel wall. The inner luminal surface may be a bare stainless steel surface or a ceramic surface, depending on the fabrication method employed, both of which are known to support endothelial cell growth. It is generally believed that, to reduce restenosis, the antiproliferative agent is needed in the first few weeks (e.g., the first 3-4 weeks) of implantation, after which release of the antiproliferative agent preferably ceases, so as not to interfere with endothelialization. In this particular embodiment, the inorganic fiber is therefore an open ended fiber, whose ends are capped with a biodegradable polymer. The biodegradable polymer is selected to degrade in a time frame such that any residual antiproliferative agent is released after the above time period has elapsed, thereby removing the agent from the site of the implant.

In other related embodiments, rather than leaving the luminal surface bare, inorganic fibers are provided on the inner luminal surface of the stent, which inorganic fibers are loaded with an endothelial cell growth promoter.

Additional advantages of the present invention include one or more of the following: (1) There is no problem with adherence of the drug delivery medium to the substrate, which may be a problem with various polymeric and non-polymeric coatings. (2) When one applies a coating directly to a substrate, one generally has to be careful that the processing does not adversely affect the substrate material. With the present invention, this is not necessary. For example, the fiber(s) can be taken to a very high temperature before loading the drug and associating the fiber(s) with a substrate. (3) In the case of a stent, the fiber can be better distributed than the cell structure of the stent, leading to more uniform drug delivery. In other words, unlike coated stents, the drug delivery area of the device is independent of the area corresponding to the stent struts. (4) In the case of a balloon deliverable stent, the inorganic fibers can be loosely wound around the stent after it has been crimped to the balloon or provided within a sheath. After expansion of the stent, the fiber is held in place between the stent and the vessel wall. (5) Where the inorganic fibers are incorporated into a biodegradable polymeric substrate, they can provide a dual function. First, the fibers can deliver a therapeutic agent into the surrounding tissue as the polymer degrades. Second, the fibers can serve as a reinforcement structure for the substrate, helping the structure stay connected as the polymer erodes. (6) Where small diameter fibers are used in conjunction with a biodegradable stent platform (e.g., a magnesium, zinc or iron stent), the fibers will exert very little significant mechanical effect on the vessel wall once the stent erodes. (7) In a design analogous to that described in Pub. No. US 2006/0184237 to Weber, one can form a mesh that contains or consists of inorganic fibers in accordance with the invention, which can be positioned around the outside surface of a self-expanding stent delivery system. After expansion of the stent, the fiber is held in place between the stent and the vessel wall. Such a system may include a self-expanding stent, a sheath adapted to enclose the self-expanding stent in an interior space during deployment, and an inorganic fiber mesh, which may be attached to the distal end self-expanding stent and which may be adapted to lay outside the interior space of the sheath when the self-expanding stent is enclosed in the sheath.

Therapeutic agents for the practice of the invention include genetic therapeutic agents and non-genetic therapeutic agents. Therapeutic agents may be used singly or in combination.

Exemplary non-genetic therapeutic agents for use in connection with the present invention include the following, among others: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan and telmisartan), and antispasmodic/anticholinergic drugs (e.g., oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Various preferred non-genetic therapeutic agents include taxanes such as paclitaxel (including particulate forms thereof, for instance, protein-bound paclitaxel particles such as albumin-bound paclitaxel nanoparticles, e.g., ABRAXANE and paclitaxel-polymer conjugates, for example, paclitaxel-poly(glutamic acid) conjugates), rapamycin (sirolimus) and its analogs (e.g., everolimus, tacrolimus, zotarolimus, etc.) as well as sirolimus-polymer conjugates and sirolimus analog-polymer conjugates such as sirolimus-poly(glutamic acid) and everolimus-poly(glutamic acid) conjugates, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Exemplary genetic therapeutic agents for use in connection with the present invention include anti-sense DNA and RNA as well as DNA coding for the various proteins (as well as the proteins themselves), for example, the following, among others: (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP), SP1017 (SUPRATEK), lipids such as cationic lipids, liposomes, lipoplexes, nanoparticles, or microparticles, with and without targeting sequences such as the protein transduction domain (PTD).

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin (sirolimus) and its analogs (e.g., everolimus, tacrolimus, zotarolimus, etc.), cerivastatin, flavopifidol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925 assigned to Kunz.

A wide range of therapeutic agent loadings can be used in conjunction with the medical devices of the present invention, with the pharmaceutically effective amount being readily determined by those of ordinary skill in the art and ultimately depending, for example, upon the condition to be treated, the nature of the therapeutic agent itself, the tissue into which the medical device is introduced, and so forth.

### EXAMPLE

Polystyrene fiber is spun onto a stainless steel stent substrate using electrospinning techniques such as those described in Lin et al. and Jarusuwannapoom et al., *supra.* The stent may be rotated while holding it at a suitable potential during electrospinning, thereby covering the stent in a meshwork of polystyrene fibers. If desired, the stent surface may be treated with an oxygen plasma prior to deposition of the fibers, in order to clean and charge the surface. To the extent that the stent windows are covered by the resulting fibrous network, they may be cleared of fiber, if desired. Alternatively, the fibrous network may be deposited on an expanded stent, in which case the fibrous network may cover then entire stent without concern that the inorganic network that is ultimately formed will inhibit expansion or be torn apart by expansion forces. The as-covered stent is then dipped into sequential polyanion and polycation solutions, for example, a first layer of PEI, followed by 7 bi-layers of PAHYPSS. The resulting structure is then washed in anhydrous alcohol (e.g., ethanol or isopropanol), followed by immersion in an anhydrous sol-gel precursor solution, for example, containing titanium isopropoxide as a metal alkoxide precursor in anhydrous alcohol, causing it to infiltrate the polyelectrolyte multilayer shell. The presence of adsorbed water in the shell then results in hydrolysis and condensation of the precursor. The resulting polyelectrolyte/sol-gel hybrid coating is then calcined at elevated temperature (e.g., 400- 600<0>C) to form a porous ceramic coating that includes a hollow ceramic fiber network. The resulting structure is then exposed to a solution of paclitaxel in DMSO (solubility ~ 500mg/ml) or in methanol (solubility ~ 100mg/ml) within an elevated pressure environment.

Further medical devices are given below:
1. A medical device comprising a substrate and a porous therapeutic-agent-loaded inorganic fiber.
2. The medical device of paragraph 1 wherein the substrate is a biostable metallic substrate.
3. The medical device of paragraph 1, wherein the substrate is a biodegradable metallic substrate.
4. The medical device of paragraph 1, wherein the substrate is a biodegradable polymeric substrate.
5. The medical device of paragraph 1, wherein the inorganic fiber is a linear fiber.
6. The medical device of paragraph 1, wherein the inorganic fiber is part of fibrous network.
7. The medical device of paragraph 1, wherein the fiber is a hollow fiber.
8. The medical device of paragraph 7, wherein the hollow fiber has closed ends.
9. The medical device of paragraph 7, wherein the hollow fiber has at least one open end.
10. The medical device of paragraph 9, wherein the open end is capped with a biodegradable material.
11. The medical device of paragraph 9, wherein the hollow fiber is loaded with a mixture comprising said therapeutic agent and a polymer.
12. The medical device of paragraph 1, wherein said inorganic fiber is a ceramic fiber that comprises a metal oxide.
13. The medical device of paragraph 1, wherein the inorganic fiber is a ceramic fiber that comprises a metal oxide selected from titanium oxide, tantalum oxide, and combinations of the foregoing.
14. The medical device of paragraph 1, wherein the inorganic fiber is nanoporous.
15. The medical device of paragraph 1, wherein said inorganic fiber is mesoporous.
16. The medical device of paragraph 1, wherein the inorganic fiber is disposed on a surface of the substrate.
17. The medical device of paragraph 1, wherein the inorganic fiber is embedded within a biodegradable polymer substrate.
18. The medical device of paragraph 1, wherein the inorganic fiber is embedded within a biodegradable polymer coating on the substrate surface.
19. The medical device of paragraph 1, wherein the inorganic fiber is wrapped around the substrate.
20. The medical device of paragraph 1, wherein the inorganic fiber is interwoven with elements of the substrate.
21. The medical device of paragraph 1, wherein the inorganic fiber is attached to the substrate.
22. The medical device of paragraph 1, wherein the inorganic fiber is not attached to substrate.
23. The medical device of paragraph 1, wherein the substrate is a tubular substrate.
24. The medical device of paragraph 23, wherein the inorganic fiber is in the form of a tubular construct that is concentric with the tubular substrate.
25. The medical device of paragraph 23, wherein the tubular substrate is a radially expandable substrate.
26. The medical device of paragraph 25, wherein the radially expandable substrate is a stent.
27. The medical device of paragraph 26, wherein the inorganic fiber is woven into the stent structure.
28. The medical device of paragraph 26, wherein the inorganic fiber is positioned over the abluminal surface of the stent.
29. The medical device of paragraph 26, wherein the inorganic fiber is formed on or applied over the stent when it is in an expanded state.
30. The medical device of paragraph 26, wherein the inorganic fiber is formed on or applied over the stent when it is in a contracted state.
31. The medical device of paragraph 30, wherein the inorganic fiber is wrapped loosely around the stent.
32. The medical device of paragraph 30, wherein the inorganic fiber is disposed longitudinally along the substrate.
33. The medical device of paragraph 30, wherein the inorganic fiber is wrapped around the substrate in a zigzag configuration.
34. The medical device of paragraph 30, wherein the inorganic fiber is part of a woven or non-woven fiber network that surrounds the stent.
35. The medical device of paragraph 1, wherein said therapeutic agent is selected from one or more of the group consisting of anti-thrombotic agents, anti-proliferative agents, anti-inflammatory agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, TGF-β elevating agents, and agents that interfere with endogenous vasoactive mechanisms.
36. The medical device of paragraph 1, wherein said inorganic fiber is a metall ic fiber that comprises a biodegradable metal selected from iron, magnesium, zinc and combinations thereof.

## Claims

1. A medical device comprising a substrate and a porous therapeutic-agent-loaded fiber, wherein said fiber is an inorganic, ceramic fiber connected to a surface of the substrate, **characterised in that** said fiber is hollow and has at least one open end that is capped with a biodegradable material.

2. The medical device of claim 1 wherein the substrate is a biostable metallic substrate or wherein the substrate is a biodegradable metallic substrate or wherein the substrate is a biodegradable polymeric substrate.

3. The medical device of claim 1, wherein the inorganic fiber is a linear fiber or wherein the inorganic fiber is pad of a fibrous network or wherein said inorganic fiber is a ceramic fiber that comprises a metal oxide or wherein the inorganic fiber is a ceramic fiber that comprises a metal oxide selected from titanium oxide, tantalum, oxide, and combinations of the foregoing.

4. The medical device of claim 1, wherein the hollow fiber is loaded with a mixture comprising said therapeutic agent and a polymer.

5. The medical device of claim 1, wherein the inorganic fiber is nanoporous or wherein said inorganic fiber is mesoporous.

6. The medical device of claim 1, wherein the inorganic fiber is disposed on a surface of the substrate or wherein the inorganic fiber is embedded within a biodegradable polymer substrate or wherein the inorganic fiber is embedded within a biodegradable polymer coating on the substrate surface or wherein the inorganic fiber is wrapped around the substrate or wherein the inorganic fiber is interwoven with elements of the substrate or wherein the inorganic fiber is attached to the substrate.

7. The medical device of claim 1, wherein the substrate is a tubular substrate.

8. The medical device of claim 7, wherein the inorganic fiber is in the form of a tubular construct that is concentric with the tubular substrate.

9. The medical device of claim 7, wherein the tubular substrate is a radially expandable substrate.

10. The medical device of claim 9, wherein the radially expandable substrate is a stent.

11. The medical device of claim 10, wherein the inorganic fiber is woven into the stent structure or wherein the inorganic fiber is positioned over the abluminal surface of the stent or wherein the inorganic fiber is formed on or applied over the stent when it is in an expanded state or wherein the inorganic fiber is formed on or applied over the stent when it is in a contracted state.

12. The medical device of claim 11, wherein the inorganic fiber is wrapped loosely around the stent or wherein the inorganic fiber is disposed longitudinally along the substrate or wherein the inorganic fiber is wrapped around the substrate in a zigzag configuration or wherein the inorganic fiber is part of a woven or non-woven fiber network that surrounds the stent.

13. The medical device of claim 1, wherein said therapeutic agent is selected from one or more of the group consisting of anti-thrombotic agents, antiproliferative agents, anti-inflammatory agents, anti-migratory agents, agents affecting extracellular matlix production and organization, antineoplastic agents, anti-mitotic agents, anesthetic agents, anti-coagulants, vascular cell growth promoters, vascular cell growth inhibitors, cholesterol-lowering agents, vasodilating agents, TGF-β elevating agents, and agents that interfere with endogenous vasoactive mechanisms.

## Patentansprüche

1. Eine medizinische Vorrichtung, aufweisend ein Substrat und eine poröse Faser, die mit einem Wirkstoff beladen ist, wobei die Faser eine anorganische Keramikfaser ist, die mit einer Oberfläche des Substrats verbunden ist, **dadurch gekennzeichnet, dass** die besagte Faser hohl ist und mindestens ein offenes Ende hat, das mit einem biologisch abbaubaren Material gedeckelt ist.

2. Die medizinische Vorrichtung gemäß dem Anspruch 1, wobei das Substrat ein biostabiles metallisches Substrat ist oder das Substrat ein biologisch abbaubares metallisches Substrat ist oder das Substrat ein biologisch abbaubares polymeres Substrat ist.

3. Die medizinische Vorrichtung gemäß dem Anspruch 1, wobei die anorganische Faser eine lineare Faser ist oder die anorganische Faser eine Einlage eines faserartigen Netzwerks ist oder die besagte anorganische Faser eine keramische Faser ist, die ein Metalloxid aufweist oder die anorganische Faser eine keramische Faser ist, die ein Metalloxid, ausgewählt aus der Gruppe aus Titanoxid, Tantaloxid oder eine Kombination der vorgenannten, aufweist.

4. Die medizinische Vorrichtung gemäß dem Anspruch 1, wobei die hohle Faser mit einer Mischung aus dem genannten Wirkstoff und einem Polymer beladen ist.

5. Die medizinische Vorrichtung gemäß dem Anspruch 1, wobei die anorganische Faser nanoporös oder mesoporös ist.

6. Die medizinische Vorrichtung gemäß dem Anspruch 1, wobei die anorganische Faser auf einer Oberfläche des Substrats angeordnet ist oder die anorganische Faser eingebettet ist in ein biologisch abbaubares Polymersubstrat oder die anorganische Faser eingebettet ist in eine biologisch abbaubare Polymerbeschichtung an der Oberfläche des Substrats oder die anorganische Faser um das Substrat herumgewickelt ist oder die anorganische Faser mit Substratelementen verflochten ist oder die anorganische Faser an dem Substrat befestigt ist.

7. Die medizinische Vorrichtung gemäß dem Anspruch 1, wobei das Substrat ein röhrenförmiges Substrat ist.

8. Die medizinische Vorrichtung gemäß dem Anspruch 7, wobei die anorganische Faser eine röhrenförmige Konstruktion aufweist, die konzentrisch zu dem röhrenförmigen Substrat angeordnet ist.

9. Die medizinische Vorrichtung gemäß dem Anspruch 7, wobei das röhrenförmige Substrat ein radial ausdehnbares Substrat ist.

10. Die medizinische Vorrichtung gemäß dem Anspruch 9, wobei das radial ausdehnbare Substrat ein Stent ist.

11. Die medizinische Vorrichtung gemäß dem Anspruch 10, wobei die anorganische Faser mit der Stent-Struktur verflochten ist oder die anorganische Faser über der abluminalen Oberfläche des Stents angeordnet ist oder die anorganische Faser an den Stent angeformt bzw. über den Stent aufgetragen ist, wenn der Stent sich in einem ausgedehnten Zustand befindet, oder die anorganische Faser an den Stent angeformt bzw. über den Stent aufgetragen ist, wenn der Stent sich in einem zusammengezogenen Zustand befindet.

12. Die medizinische Vorrichtung gemäß dem Anspruch 11, wobei die anorganische Faser locker um den Stent gewickelt ist oder die anorganische Faser entlang der Längsrichtung des Substrats angeordnet ist oder die anorganische Faser in einer Zickzack-Anordnung um das Substrat gewickelt ist oder die anorganische Faser Teil eines verflochtenen oder nicht verflochtenen Fasernetzwerks ist, welches den Stent umgibt.

13. Die medizinische Vorrichtung gemäß dem Anspruch 1, wobei der Wirkstoff ausgewählt ist aus einem oder mehreren aus der Gruppe bestehend aus antithrombotische Mittel, antiproliferative Mittel, entzündungshemmende Mittel, migrationshämmende Mittel, Mittel, die die extrazelluläre Matrixproduktion und -organisation beeinflussen, antineoplastische Mittel, antimitotische Mittel, Betäubungsmittel, Gerinnungshämmer, Promotoren für das Gefäßzellwachstum, Inhibitoren für das Gefäßzellwachstum, cholesterinsenkende Mittel, gefäßerweiternde Mittel, TGF-β anhebende Mittel und Mittel, die in die endogenen vasoaktiven Mechanismen eingereifen.

## Revendications

1. Dispositif médical comprenant un substrat et une fibre poreuse chargée d'agents thérapeutiques, dans lequel ladite fibre est une fibre céramique inorganique rattachée à une surface du substrat, **caractérisé en ce que** ladite fibre est creuse et présente au moins une extrémité ouverte qui est recouverte d'un matériau biodégradable.

2. Dispositif médical selon la revendication 1, dans lequel le substrat est un substrat métallique biostable ou dans lequel le substrat est un substrat métallique biodégradable ou dans lequel le substrat est un substrat polymère biodégradable.

3. Dispositif médical selon la revendication 1, dans lequel la fibre inorganique est une fibre linéaire ou dans lequel la fibre inorganique est un tampon d'un réseau fibreux ou dans lequel ladite fibre inorganique est une fibre céramique qui comprend un oxyde métallique ou dans lequel la fibre inorganique est une fibre céramique qui comprend un oxyde métallique sélectionné dans le groupe comprenant l'oxyde de titane, l'oxyde de tantale et des combinaisons de ceux-ci.

4. Dispositif médical selon la revendication 1, dans lequel la fibre creuse est chargée d'un mélange comprenant ledit agent thérapeutique et un polymère.

5. Dispositif médical selon la revendication 1, dans lequel la fibre inorganique est nanoporeuse ou dans lequel ladite fibre inorganique est mésoporeuse.

6. Dispositif médical selon la revendication 1, dans lequel la fibre inorganique est placée sur une surface du substrat ou dans lequel la fibre inorganique est noyée dans un substrat polymère biodégradable ou dans lequel la fibre inorganique est noyée dans un revêtement polymère biodégradable sur la surface de substrat ou dans lequel la fibre inorganique est enroulée autour du substrat ou dans lequel la fibre inorganique est entrecroisée avec des éléments du substrat ou dans lequel la fibre inorganique est fixée au substrat.

7. Dispositif médical selon la revendication 1, dans lequel le substrat est un substrat tubulaire.

8. Dispositif médical selon la revendication 7, dans lequel la fibre inorganique a la forme d'une construction tubulaire qui est concentrique avec le substrat tubulaire.

9. Dispositif médical selon la revendication 7, dans lequel le substrat tubulaire est un substrat radialement extensible.

10. Dispositif médical selon la revendication 9, dans lequel le substrat radialement extensible est un stent.

11. Dispositif médical selon la revendication 10, dans lequel la fibre inorganique est tissée dans la structure de stent ou dans lequel la fibre inorganique est positionnée par-dessus la surface abluminale du stent ou dans lequel la fibre inorganique est formée sur le stent ou appliquée par-dessus celui-ci lorsqu'il se trouve dans un état dilaté ou dans lequel la fibre inorganique est formée sur le stent ou appliquée par-dessus celui-ci lorsqu'il se trouve dans un état contracté.

12. Dispositif médical selon la revendication 11, dans lequel la fibre inorganique est enroulée avec du jeu autour du stent ou dans lequel la fibre inorganique est placée longitudinalement le long du substrat ou dans lequel la fibre inorganique est enroulée autour du substrat dans une configuration en zigzag ou dans lequel la fibre inorganique fait partie d'un réseau de fibres tissées ou non-tissées qui entoure le stent.

13. Dispositif médical selon la revendication 1, dans lequel ledit agent thérapeutique est sélectionné dans un ou plusieurs du groupe constitué par des agents antithrombotiques, des agents antiprolifératifs, des agents anti-inflammatoires, des agents antimigrations, des agents affectant la production et l'organisation des matrices extracellulaires, des agents antinéoplastiques, des agents antimitotiques, des agents anesthésiants, des anticoagulants, des promoteurs de croissance cellulaire vasculaire, des inhibiteurs de croissance cellulaire vasculaire, des agents d'abaissement du taux de cholestérol, des agents vasodilatateurs, des agents élevant le TGF-β, et des agents qui interfèrent avec des mécanismes vasoactifs endogènes.
